# EUROPÄISCHE PATENTSCHRIFT

(11) **EP 0 779 265 B1**
(45) Veröffentlichungstag und Bekanntmachung des Hinweises auf die Patenterteilung: **23.08.2000**
(21) Anmeldenummer: 96119264.8
(22) Anmeldetag: 02.12.1996
(51) Int. Cl.: C07C 53/16, C07C 51/487, C07C 51/43

(54) **Verfahren zur Darstellung einer besonders reinen Monochloressigsäure**
Process for preparing particularly pure monochloroacetic acid
Procédé de préparation d'acide monochloroacétique particulièrement pur

(30) Priorität: 11.12.1995 DE 19546080
(43) Veröffentlichungstag der Anmeldung: 18.06.1997
(73) Patentinhaber: Clariant GmbH, 65929 Frankfurt am Main (DE)
(72) Erfinder: Ebmeyer, Frank, Dr., 86153 Augsburg (DE); Kampmann, Detlef, Dr., 86368 Gersthofen (DE); Paulus-Von Russdorf, Ulf Otto, 86368 Gersthofen (DE); Rossmeissl, Rudolf, 86690 Mertingen (DE)

(56) Entgegenhaltungen:
- DE-A- 4 313 121
- GERHARTZ W. & YAMAMOTO Y.S.: "ULLMANN'S ENCYCLOPEDIA OF INDUSTRIAL CHEMISTRY, FIFTH EDITION, VOL. A 6" 1985 , VCH VERLAG , WEINHEIM XP002027640 173110 "CHLOROACETIC ACIDS" * Seite 537 - Seite 552 * * Seite 540, Spalte 1, Zeile 25 - Spalte 2, Zeile 20 *

## Beschreibung

Die vorliegende Erfindung betrifft ein Verfahren zur Darstellung einer besonders reinen Monochloressigsäure, wie sie zur Synthese von Grundchemikalien der pharmazeutischen Industrie und der Kosmetikindustrie benötigt wird.

Monochloressigsäure wird durch direkte Chlorierung von Essigsäure hergestellt. Hierbei entsteht durch Überchlorierung als Zwangsanfall das unerwünschte Nebenprodukt Dichloressigsäure, welches durch Destillation, Kristallisation oder Hydrierung entfernt werden kann.

Aufgrund der sehr ähnlichen Siedepunkte (Monochloressigsäure: 189°C; Dichloressigsäure: 194°C) ist eine destillative Abtrennung aufwendig und unökonomisch.

Bekannt ist die Entfernung von Dichloressigsäure aus der nach der Chlorierung erhaltenen rohen Monochloressigsäure durch Hydrierung an einem heterogenen Katalysator (z. B. nach EP-A-453 690, DE-A-1915037, EP-A-537838). Soll die gewöhnlich in einer Konzentration von 2 - 5 Gew.-% der rohen Monochloressigsäure anhaftende Dichloressigsäure auf eine Konzentration <100 ppm erniedrigt werden, so erfordert dies lange Verweilzeiten der rohen Monochloressigsäure in den Hydrierreaktoren bzw. große Katalysatorvolumina, d.h. das Verfahren wird technisch aufwendig und umständlich. Außerdem tritt bei einer solchen Verfahrensweise die als Nebenreaktion unerwünschte Hydrierung von Monochloressigsäure zu Essigsäure verstärkt ein. Technisch sinnvoll ist es, die Hydrierung so zu betreiben, daß eine Konzentrationsabnahme der Dichloressigsäure um den Faktor 10, also beispielsweise von 3 auf 0,3 Gew.-%, in Ausnahmefällen bei Verwendung sehr selektiver Katalysatoren auch um den Faktor 100, also beispielsweise von 3 auf 0,03 Gew.-%, erzielt wird. Diese Vorgehensweise wird in obigen Druckschriften beschrieben.

Weiterhin bekannt ist die Entfernung von Dichloressigsäure aus der rohen Monochloressigsäure durch Schmelzkristallisation (Ullmanns Enzyklopädie der technischen Chemie, 4. Auflage, 1975, Bd. 9, S. 395). Hierbei gelingt mit einer einstufigen Umkristallisation eine Konzentrationsabnahme der Dichloressigsäure um den Faktor 4, also beispielsweise von 3 auf 0,7 - 0,8 Gew.-%. Der Platz und Zeitbedarf für dieses Verfahren ist jedoch erheblich. Außerdem verbleibt nach mehrmaliger Kristallisation ein Gemisch aus Monochloressigsäure und Dichloressigsäure als Mutterlauge, die je nach den Bedingungen der Auskühlung noch mindestens 30 Gew.-% Monochloressigsäure enthält, die durch weitere Kristallisation nicht in ein verkaufsfähiges Produkt umgewandelt werden kann.

Bekannt ist auch die Entfernung von Dichloressigsäure, da entweder die gesamte Rohsäure oder die Mutterlauge nach Kristallisation noch hydrierdt werden können (Ullmanns Enzyklopädie der technischen Chemie, S. Auflage, 1985, S. 537-552).

Es bestand somit die Aufgabe, ein Verfahren zur Herstellung einer besonders reinen Monochloressigsäure zur Verfügung zu stellen, das die oben beschriebenen Nachteile nicht aufweist.

Überraschenderweise wurde nun ein Verfahren zur Herstellung einer besonders reinen Monochloressigsäure gefunden, bei dem in einem ersten Schritt eine Hydrierung eines Gemisches aus Mono- und Dichloressigsäure bis zu einem Restgehalt an Dichloressigsäure von 400 bis 600 ppm durchgeführt wird und wobei in einem zweiten Schritt dieses Gemisch einer Schmelzkristallisation unterworfen wird.

Das erfindungsgemäße Verfahren erlaubt mit geringem technischen Aufwand eine sehr ökonomische Entfernung der in der rohen Monochloressigsäure anfallenden Dichloressigsäure. Durch selektive Hydrierung der Dichloressigsäure wird die unerwünschte Bildung von Essigsäure hierbei ebenso erfolgreich vermieden wie der Anfall von hochkonzentrierter Mutterlauge.

Besonders überraschend war, daß sich durch Schmelzkristallisation auch bei Gemischen mit weniger als 400 ppm Dichloressigsäure noch eine deutliche Konzentrationsabnahme erzielen ließ. Normalerweise werden nur deutlich höher konzentrierte Gemische einer Kristallisation unterworfen.

Für die Hydrierung wird ein heterogener Katalysator verwendet, welcher 0,1 - 5,0, bevorzugt 0,3 - 4 Gew.-% Palladium enthält und Aktivkohle oder Siliciumdioxid als Träger verwendet. Die BET-Oberfläche beträgt 100 - 1300, bevorzugt 500 - 1200 m²/g.

Die Hydrierung wird bei einem Überdruck von 0 - 10 bar, insbesondere von 0 - 4 bar und bei einer Temperatur von 100 - 200°C, insbesondere von 110 - 150°C durchgeführt.

Das erfindungsgemäße Verfahren ist auch geeignet für Gemische mit einem sehr hohen Anteil an Dichloressigsäure. So kann selbst ein eutektisches Gemisch aus Monochloressigsäure und 49 Gew.-% Dichloressigsäure problemlos gereinigt werden (siehe Beispiel 3).

Die Kristallisation wird als Schmelzkristallisation ohne Verwendung eines Lösungsmittels durchgeführt. In einem ersten Schritt wird zunächst durch Aufheizen eine Schmelze erzeugt, die dann in einem zweiten Schritt durch Abkühlen zum Auskristallisieren gebracht wird. Der Kristallisationsprozeß wird dabei so durchgeführt, daß Dichloressigsäure und andere flüssige oder gelöste Verunreinigungen in der Restschmelze verbleiben und mit ihr entfernt werden können. Man kann damit beispielsweise Monochloressigsäure mit einem Restgehalt von nur noch 80 ppm an Dichloressigsäure erhalten.

Die folgenden Beispiele sollen die Erfindung erläutern, ohne sie zu beschränken:

### Beispiel 1

2 kg/h Monochloressigsäure mit einem Dichloressigsäuregehalt von 3,2 Gew.-% werden zusammen mit 800 Nl/h Wasserstoff bei einem Überdruck von 1 bar und einer Temperatur von 130°C über ein sich in einem Rohrreaktor befindendes Katalysatorfestbett mit einem Volumen von 2 l geleitet. Als Katalysator wird eine Aktivkohle mit 0,5 Gew.-% Pd verwendet. Die BET-Oberfläche beträgt 1000 m²/g. Das Produkt dieser Reaktion, welches 0,6 Gew.-% Dichloressigsäure enthält, wird in einen Kristallisator geleitet und dort einer einmaligen Schmelzkristallisation unterworfen. Nach diesem einmaligen Umkristallisieren enthält die Monochloressigsäure 0,15 Gew.-% Dichloressigsäure. Das Produkt ist farblos und im geschmolzenen Zustand wasserklar.

### Beispiel 2

Darstellung einer besonders reinen Monochloressigsäure:
Man verfährt wie in Beispiel 1, senkt jedoch den Durchsatz an Monochloressigsäure in der Hydrierung auf 1 kg/h. Daraufhin erhält man ein Hydrierprodukt, welches 0,035 Gew.-% Dichloressigsäure enthält. Nach der Umkristallisation findet man lediglich noch 0,008 Gew.-% Dichloressigsäure in der Monochloressigsäure. Das Produkt ist ebenfalls farblos und im geschmolzenen Zustand wasserklar.

### Beispiel 3

Man verfährt wie in Beispiel 2, verwendet jedoch als Edukt eine Monochloressigsäure, welche 49 Gew.-% Dichloressigsäure enthält und senkt den Durchsatz auf 0,5 kg/h. Nach Hydrierung verbleibt ein Produkt mit einem Dichloressigsäuregehalt von 8 Gew.-%. Das Produkt der Umkristallisation enthält noch 2,2 Gew.-% Dichloressigsäure, ist farblos und im geschmolzenen Zustand wasserklar. Mit diesen Eigenschaften ist es hervorragend geeignet als Edukt für die in den Beispielen 1 und 2 beschriebenen Verfahrensweisen. Auf diese Weise läßt sich auch aus dem eutektischen Gemisch von Monochloressigsäure und Dichloressigsäure eine Monochloressigsäure hoher Qualität herstellen.

### Beispiel 4

400 kg/h Monochloressigsäure mit einem Dichloressigsäuregehalt von 3,1 Gew.-% werden zusammen mit 15 Nm³/h Wasserstoff bei einem Überdruck von 1 bar und einer Temperatur von 130°C über ein sich in einem Rohrreaktor befindenden Katalysatorfestbett mit einem Volumen von 800 l geleitet. Als Katalysator wird eine Aktivkohle mit 0,5 Gew.-% Pd verwendet. Das Hydrierprodukt enthält 0,040 Gew.-% Dichloressigsäure. Nach Umkristallisation enthält die Monochloressigsäure lediglich noch 0,010 Gew.-% Dichloressigsäure. Das Produkt ist farblos und im geschmolzenen Zustand wasserklar.

### Beispiel 5

1 kg/h Monochloressigsäure mit einem Dichloressigsäuregehalt von 22 Gew.-% werden zusammen mit 2600 Nl/h Wasserstoff bei einem Überdruck von 4 bar und einer Temperatur von 130°C über ein sich in einem Rohrreaktor befindendes Katalysatorfestbett mit einem Volumen von 2 l geleitet. Als Katalysator wird eine Aktivkohle mit 1,0 Gew.% Pd verwendet. Die BET-Oberfläche beträgt 1000 m²/g. Das Produkt dieser Reaktion, welches 1,0 Gew.-% Dichloressigsäure enthält, wird in einen Kristallisator geleitet und dort einer einmaligen Schmelzkristallisation unterworfen. Nach diesem einmaligen Umkristallisieren enthält die Monochloressigsäure 0,25 Gew.-% Dichloressigsäure. Das Produkt ist farblos und im geschmolzenen Zustand wasserklar.

### Beispiel 6

Man verfährt wie in Beispiel 5, senkt jedoch den Durchsatz an Monochloressigsäure in der Hydrierung auf 0,5 kg/h. Daraufhin erhält man ein Hydrierprodukt, welches 0,16 Gew.-% Dichloressigsäure enthält. Nach der Umkristallisation findet man lediglich noch 0,04 Gew.-% Dichloressigsäure in der Monochloressigsäure. Das Produkt ist ebenfalls farblos und im geschmolzenen Zustand wasserklar.

## Patentansprüche

1. Verfahren zur Darstellung einer Monochloressigsäure mit nur noch 0,008 Gew.-% Dichloressigsäure, dadurch gekennzeichnet, daß in einem ersten Schritt eine Hydrierung eines Gemisches aus Mono- und Dichloressigsäure bis zu einem Restgehalt an Dichloressigsäure von 400 bis 600 ppm durchgeführt wird und daß in einem zweiten Schritt dieses Gemisch einer Schmelzkristallisation unterworfen wird.

2. Verfahren nach Anspruch 1, dadurch gekennzeichnet, daß die Hydrierung mit einem heterogenen Katalysator durchgeführt wird, welcher 0,1 - 5,0 Gew.-% Palladium enthält und bei dem als Trägermaterial Aktivkohle oder Siliciumdioxid verwendet wird.

3. Verfahren nach Anspruch 1 oder 2, dadurch gekennzeichnet, daß der Katalysator 0,3 - 4,0 Gew.-% Palladium enthält.

4. Verfahren nach mindestens einem der Ansprüche 1 bis 3, dadurch gekennzeichnet, daß die Hydrierung bei einem Überdruck von 0 - 10 bar durchgeführt wird.

5. Verfahren nach mindestens einem der Ansprüche 1 bis 3, dadurch gekennzeichnet, daß die Hydrierung bei einem Überdruck von 0 - 4 bar durchgeführt wird.

6. Verfahren nach mindestens einem der Ansprüche 1 bis 5, dadurch gekennzeichnet, daß die Hydrierung bei einer Temperatur von 100 - 200°C durchgeführt wird.

7. Verfahren nach mindestens einem der Ansprüche 1 bis 5, dadurch gekennzeichnet, daß die Hydrierung bei einer Temperatur von 110 - 150°C durchgeführt wird.

8. Verfahren nach mindestens einem der Ansprüche 1 bis 7, dadurch gekennzeichnet, daß als Edukt für das Verfahren eine Monochloressigsäure benutzt wird, die 1 - 50 Gew.-% Dichloressigsäure enthält.

9. Verfahren nach Anspruch 8, dadurch gekennzeichnet, daß als Edukt eine Monochloressigsäure mit einem Dichloressigsäuregehalt von 20 - 50 Gew.-% eingesetzt wird und daß die Hydrierung vor der Schmelzkristallisation zweimal durchlaufen wird.

## Claims

1. A process for the preparation of a monochloroacetic acid comprising only 0.008% by weight of dichloroacetic acid, which comprises, in a first step, carrying out a hydrogenation of a mixture of mono- and dichloroacetic acid to a residual dichloroacetic acid content of 400 to 600 ppm and, in a second step, subjecting this mixture to melt crystallization.

2. The process as claimed in claim 1, wherein the hydrogenation is carried out with a heterogeneous catalyst which comprises 0.1 - 5.0% by weight of palladium and in which active charcoal or silicon dioxide is used as the support.

3. The process as claimed in claim 1 or 2, wherein the catalyst comprises 0.3 - 4.0% by weight of palladium.

4. The process as claimed in at least one of claims 1 to 3, wherein the hydrogenation is carried out under an increased pressure of 0 - 10 bar.

5. The process as claimed in at least one of claims 1 to 3, wherein the hydrogenation is carried out under an increased pressure of 0 - 4 bar.

6. The process as claimed in at least one of claims 1 to 5, wherein the hydrogenation is carried out at a temperature of 100 - 200°C.

7. The process as claimed in at least one of claims 1 to 5, wherein the hydrogenation is carried out at a temperature of 110 - 150°C.

8. The process as claimed in at least one of claims 1 to 7, wherein a monochloroacetic acid which comprises 1 - 50% by weight of dichloroacetic acid is used as the starting material for the process.

9. The process as claimed in claim 8, wherein a monochloroacetic acid having a dichloroacetic acid content of 20 - 50% by weight is employed as the starting material and the hydrogenation is passed through twice before the melt crystallization.

## Revendications

1. Procédé de préparation d'un acide monochloracétique ne présentant qu'une teneur de 0,008% massiques en acide dichloracétique, caractérisé en ce qu'on réalise, dans une première étape, une hydrogénation d'un mélange d'acides monochloracétique et dichloracétique jusqu'à ce que la teneur résiduelle en acide dichloracétique soit de 400 à 600 ppm, et dans une seconde étape, une cristallisation par fusion de ce mélange.

2. Procédé selon la revendication 1, caractérisé en ce qu'on met en oeuvre l'hydrogénation sur un catalyseur hétérogène, lequel présente une teneur de 0,1 à 5,0% massiques en palladium et dont le support est un charbon actif ou le dioxyde de silicium.

3. Procédé selon la revendication 1 ou 2, caractérisé en ce que le catalyseur présente une teneur de 0,3 à 4,0% massiques en palladium.

4. Procédé selon au moins l'une des revendications 1 ou 3, caractérisé en ce qu'on met en oeuvre l'hydrogénation à une surpression de 0 à 10 bars.

5. Procédé selon au moins l'une des revendications 1 ou 3, caractérisé en ce qu'on met en oeuvre l'hydrogénation à une surpression de 0 à 4 bars.

6. Procédé selon au moins l'une des revendications 1 à 5, caractérisé en ce qu'on met en oeuvre l'hydrogénation à une température de 100 à 200°C.

7. Procédé selon au moins l'une des revendications 1 à 5, caractérisé en ce qu'on met en oeuvre l'hydrogénation à une température de 110 à 150°C.

8. Procédé selon au moins l'une des revendications 1 à 7, caractérisé en ce qu'on utilise comme matière de départ dans le procédé un acide monochloracétique présentant une teneur de 1 à 50% massiques en acide dichloracétique.

9. Procédé selon la revendication 8, caractérisé en ce qu'on utilise comme matière de départ un acide monochloracétique ayant une teneur de 20 à 50% massiques en acide dichloracétique et qu'on réalise l'hydrogénation deux fois avant la cristallisation par fusion.
